# EUROPEAN PATENT SPECIFICATION

(11) **EP 1 058 687 B1**
(45) Date of publication and mention of the grant of the patent: **10.12.2003**
(21) Application number: 99932499.9
(22) Date of filing: 12.02.1999
(51) Int. Cl.: C07H 19/16, C07H 19/167, A61K 31/70, C07K 5/00

(54) **NUCLEOSIDES**
NUCLEOSIDE
NUCLEOSIDES

(30) Priority: 13.02.1998 SE 9800452; 13.08.1998 ZA 9807267; 14.08.1998 WO PCT/SE98/01467
(43) Date of publication of application: 13.12.2000
(73) Proprietor: MEDIVIR AB, 141 44 Huddinge (SE)
(72) Inventor: WÄHLING, Horst, S-127 61 Skärholmen (SE); ZHOU, Xiao-Xiong, S-141 41 Huddinge (SE)
(74) Representative: Dehmel, Albrecht, Dr.
(86) International application number: SE9900189
(87) International publication number: WO99041268

(56) References cited:
- WO-A1-88/00050
- WO-A1-92/13561
- WO-A1-94/24134
- US-A- 5 543 414

## Description

### Technical Field

This invention relates to the field of nucleoside analogues, such as antivirals including inhibitors of retroviral reverse transcriptase and the DNA polymerase of Hepatitis B Virus (HBV). The invention provides novel compounds with favourable pharmaceutical parameters, methods for their preparation, pharmaceutical compositions comprising these compounds and methods employing them for the inhibition of viral and neoplastic diseases including HBV and HIV.

### Background to the invention

International patent application WO 88/00050 describes the antiretroviral and anti-HBV activity of a series of 3'-fluorinated nucleosides, including the compounds 2',3'-dideoxy, 3'-fluoroguanosine (FLG) and 3'-fluorothymidine (FLT). The latter compound underwent clinical evaluation as an anti-HIV agent and although its antiviral activity and pharmacokinetics were good, it showed unexpected toxicity (Flexner et al, J Inf Dis 170(6) 1394-403 (1994)). The former compound FLG is very active in vitro. However, the present inventors have detected that its bioavailability is so poor - around 4% - that the in vivo utility of the compound has thus far been limited to intraperitoneally or subcutaneously administered animal models.

US patent 4,963,662 discloses generically a series of 3'-fluorinated nucleosides and corresponding triphosphates and specifically describes the preparation of the 5'-O-palmitoyl derivative of FLT, without reporting any improvement in bioavailability. International patent application WO 93/13778 describes FLG derivatives modified at the 6-position of the base, in particular with n-propoxy, cyclobutoxy, cyclopropanylamino, piperidino or pyrrolidino. International patent application WO 93/14103 describes FLG derivatives where the oxygen at the guanine 6-position is replaced with amino, ether, halo or sulphonate.

Our patent applications PCT/SE 98/01467 and TH 45550 (published as WO 99/09031 and TH 39363, respectively) describe prodrugs of FLG comprising a linker structure and one or two aliphatic amino acids. We have now discovered a particularly convenient prodrug within the broad concept disclosed in these patent applications which produces superior bioavailability, while at the same time resulting in metabolic degradation products which are nature identical. In other words, the metabolic by-products are identical to compounds produced by the body for which the body has efficient regulatory and elimination machinery.

### Brief description of the invention

In accordance with the invention there are provided compounds of the formula I: 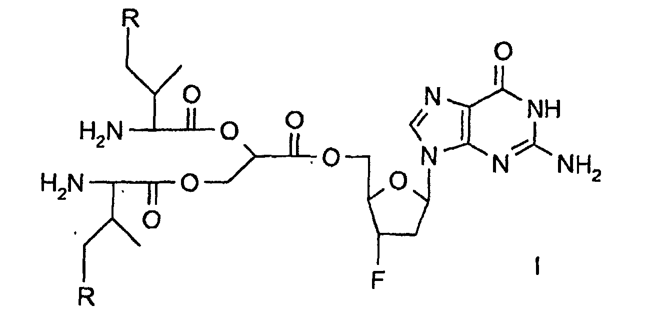 wherein R is independently H or -CH₃;
and pharmaceutically acceptable salts thereof.

The invention further provides pharmaceutical compositions comprising the compounds and salts of formula I and pharmaceutically acceptable carriers or diluents therefor. The compositions of the invention are useful in methods for the inhibition of HBV and retroviruses such as HIV, for example by administering an effective amount of the compound or salt to an individual afflicted with a retrovirus or HBV. The invention also extends to the use of the compounds or salts of formula I in therapy, for example in the preparation of a medicament for the treatment of retroviral or HBV infections.

In treating conditions caused by retroviruses such as HIV, or HBV, the compounds or salts of formula I are preferably administered in an amount of 50 to 1 500 mg once, twice or three times per day, especially 100 to 700 mg twice or thrice daily. It is desirable to achieve serum levels of the active metabolite of 0.01 to 100 µg/ml, especially 0.1 to 5 µg/ml.

Thus preferred compounds of formula I include:
5'-O-[(S,R) 2,3-bis-(L-valyloxy)-propionyl]-2',3'-dideoxy-3'-fluoroguanosine,
5'-O-[(S,R) 2,3-bis-(L-isoleucyloxy)-propionyl]-2',3'-dideoxy-3'-fluoroguanosine,
and most preferably
5'-O-[(R) 2,3-bis-(L-valyloxy)-propionyl]-2',3'-dideoxy-3'-fluoroguanosine,
5'-O-[(R) 2,3-bis-(L-isoleucyloxy)-propionyl]-2',3'-dideoxy-3-fluoroguanosine;
and their pharmaceutically acceptable salts.

The compounds of the invention can form salts which form an additional aspect of the invention. Appropriate pharmaceutically acceptable salts of the compounds of Formula I include salts of organic acids, especially carboxylic acids, including but not limited to acetate, trifluoroacetate, lactate, gluconate, citrate, tartrate, maleate, malate, pantothenate, isethionate, adipate, alginate, aspartate, benzoate, butyrate, digluconate, cyclopentanate, glucoheptanate, glycerophosphate, oxalate, heptanoate, hexanoate, fumarate, nicotinate, palmoate, pectinate, 3-phenylpropionate, picrate, pivalate, proprionate, tartrate, lactobionate, pivolate, camphorate, undecanoate and succinate, organic sulphonic acids such as methanesulphonate, ethanesulphonate, 2-hydroxyethane sulphonate, camphorsulphonate, 2-napthalenesulphonate, benzenesulphonate, p-chlorobenzenesulphonate and p-toluenesulphonate; and inorganic acids such as hydrochloride, hydrobromide, hydroiodide, sulphate, bisulphate, hemisulphate, thiocyanate, persulphate, phosphoric and sulphonic acids. The compounds of Formula I may in some cases be isolated as the hydrate.

In keeping with the usual practice with retroviral and HBV inhibitors it is advantageous to co-administer one to three or more additional antivirals, such as AZT (zidovudine), ddI (didanosine), ddC (zalcitabine), d4T (stavudine), 3TC (lamivudine), H2G (omaciclovir), abacavir, ABT 606 (valomaciclovir), foscarnet, ritonavir, indinavir, saquinavir, nevirapine, delaviridine, efavirenz, Vertex VX 478 (amprenavir) or Agouron AG1343 (nelfinavir) in the case of HIV or lamivudine, interferon, famciclovir, adefovir, lobucovir, BMS 200475 (entecavir), L-FMAU (clevudine), FTC (emtricitabine), DAPD (amdoxovir) in the case of HBV. Such additional antivirals will normally be administered at dosages relative to each other which broadly reflect their respective therapeutic values. Molar ratios of 100:1 to 1:100, especially 25:1 to 1:25, relative to the compound or salt of formula I will often be convenient. Administration of additional antivirals is generally less common with those antiviral nucleosides intended for treating herpes infections.

While it is possible for the active agent to be administered alone, it is preferable to present it as part of a pharmaceutical formulation. Such a formulation will comprise the above defined active agent together with one or more acceptable carriers/excipients and optionally other therapeutic ingredients. The carrier(s) must be acceptable in the sense of being compatible with the other ingredients of the formulation and not deleterious to the recipient.

The formulations include those suitable for rectal, nasal, topical (including buccal and sublingual), vaginal or parenteral (including subcutaneous, intramuscular, intravenous and intradermal) administration, but preferably the formulation is an orally administered formulation. The formulations may conveniently be presented in unit dosage form, e.g. tablets and sustained release capsules, and may be prepared by any methods well known in the art of pharmacy.

Such methods include the step of bringing into association the above defined active agent with the carrier. In general, the formulations are prepared by uniformly and intimately bringing into association the active agent with liquid carriers or finely divided solid carriers or both, and then if necessary shaping the product. The invention extends to methods for preparing a pharmaceutical composition comprising bringing a compound of Formula I or its pharmaceutically acceptable salt in conjunction or association with a pharmaceutically acceptable carrier or vehicle. If the manufacture of pharmaceutical formulations involves intimate mixing of pharmaceutical excipients and the active ingredient in salt form, then it is often preferred to use excipients which are non-basic in nature, i.e. either acidic or neutral.

Formulations for oral administration in the present invention may be presented as discrete units such as capsules, cachets or tablets each containing a predetermined amount of the active agent; as a powder or granules; as a solution or a suspension of the active agent in an aqueous liquid or a non-aqueous liquid; or as an oil-in-water liquid emulsion or a water in oil liquid emulsion and as a bolus.

With regard to compositions for oral administration (e.g. tablets and capsules), the term "suitable carrier" includes vehicles such as common excipients, e.g., binding agents, for example syrup, acacia, gelatin, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example com starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavouring agents such as peppermint, oil of wintergreen and cherry flavouring can also be used. It may be desirable to add a colouring agent to make the dosage form readily identifiable. Tablets may also be coated by methods well known in the art.
A tablet may be made by compression or moulding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

A still further aspect of the invention provides a method for the preparation of a compound of 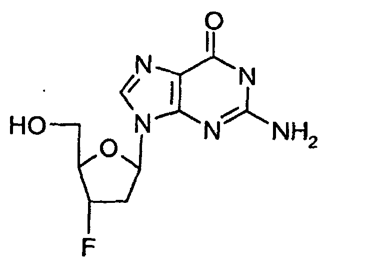 Formula I comprising the acylation of the nucleoside FLG,
with an activated acid of the formula: 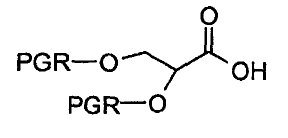 where PGR is R (as defined above) N-protected with a conventional protecting group such as Fmoc, BOC or CBz.

The activated derivative used in the acylation may comprise, e.g,, the acid halide, acid anhydride, activated acid ester or the acid in the presence of coupling reagent, for example dicyclohexylcarbodiimide. Representative activated acid derivatives include the acid chloride, anhydrides derived from alkoxycarbonyl halides such as isobutyloxycarbonylchloride, N-hydroxysuccinamide derived esters, N-hydroxyphthalimide derived esters, N-hydroxy-5-norbomene-2,3-dicarboxamide derived esters and 2,4,5-trichlorophenol derived esters. Further activated acids include those where X in the formula RX represents an OR'moiety where R is R as defined herein, and R' is, for example COCH₃, COCH₂CH₃ or COCF₃, or where X is benzotriazole.

The activated acid may be pre-formed or generated in situ by the use of reagents such as dicyclohexylcarbodiimide (DCC) or O-(1H-benzotriazol-1-yl) N,N,N',N'-tetramethyluronium tetrafluoroborate (TBTU). When an acid halide, such as the acid chloride is used, a tertiary amine catalyst, such as triethylamine, N,N'-dimethylaniline, pyridine or dimethylaminopyridine may be added to the reaction mixture to bind the liberated hydrohalic acid.

The reactions are preferably carried out in an unreactive solvent such as N,N-dimethylformamide, tetrahydrofuran, dioxane, acetonitrile or a halogenatated hydrocarbon, such as dichloromethane. If desired, any of the above mentioned tertiary amine catalysts may be used as solvent, taking care that a suitable excess is present. The reaction temperature can typically be varied between 0°C and 60°C, but will preferably be kept between 5°C and 50°C. After a period of 1 to 60 hours the reaction will usually be essentially complete. The progress of the reaction can be followed using thin layer chromatography (TLC) and appropriate solvent systems. In general, when the reaction is completed as determined by TLC, the product is extracted with an organic solvent and purified by chromatography and/or recrystallisation from an appropriate solvent system.

By-products where acylation has taken place on the nucleoside base can be separated by chromatography, but such misacylation can be minimized by controlled reaction conditions. These controlled conditions can be achieved, for example, by manipulating the reagent concentrations or rate of addition, especially of the acylating agent, by lowering the temperature or by the choice of solvent. The reaction can be followed by TLC to monitor the controlled conditions. It may be convenient to protect the 6-oxo group on the base and especially the 2-amino with conventional protecting groups to forestall misacylation.

The 2,3-bis N-protected amino acyl glyceric acid intermediate is prepared by protecting the carboxy group of glyceric acid with a conventional protecting group such as methoxybenzyl and esterifying with the appropriate N-protected amino acid, followed by selective deprotection of the methoxybenzyl group.

The amino acid derivative of R₂ and, if present, R₁ can alternatively be esterified to the linker group with the 2-oxa-4-aza-cycloalkane-1,3-dione methodology described in international patent application WO 94/29311.

Detailed description

### EXAMPLE 1

### 2,3-Bis-(N-CBz-L-valyloxy)-propionic acid.(MSS-137)

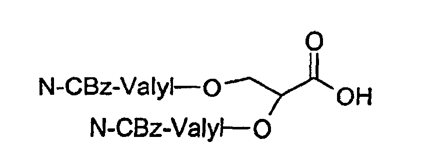

a) 1,1-dimethylethyl-2,3-(bis N-CBZ-L-valyloxy)propionate
   To a solution of 1,1-dimethylethyl-2,3-dihydroxypropionate (2.43g, 15 mmole), N-CBZ-L-valine (7.54g, 30 mmole) and DMAP (0.37g, 3 mmole) in 150 ml dichloromethane was added DCC (7.2g 35 mmole) and the mixture was stirred for two days at room temperature. The mixture was cooled to about 5°C and the urethane was filtered. The filtrate was evaporated, ethyl acetate was added and the organic phase washed twice with 5% acetic acid, 5% sodium hydrogen carbonate and water. The organic phase was dried with sodium sulfate, filtered and evaporated under reduced pressure. The product was isolated by silica gel column chromatography. Yield: 8.2g = 86%
   ¹H-NMR (DMSO d-6) 0.87 (m, 12H) 1.40 (d, 9H) 2.12 (m, 1H) 4.02-4.40 (m, 2H) 5.04 (d, 4H) 5.20 (m, 1H) 7.36 (m, 10H) 7.72 (d, 2H)
b) 2,3-Bis-(N-CBZ-L-valyloxy)-propionic acid.
   To a solution of 1,1-dimethylethyl-2,3-bis-(N-CBZ-L-valyloxy)-propionate (7.2g, 11.4 mmole) in dichloromethane (25 ml) was added trifluoroacetic acid (25 ml) and the solution was stirred for five hours at room temperature. The solution was evaporated under reduced pressure and coevaporated two times with toluene. The product was isolated by silica gel column chromatography. Yield : 5.9g = 90%
   ¹H-NMR (DMSO-d6) 0.92 (m, 12H) 2.08 (m, 2H) 3.92-4.17 (m, 2H) 4.30-4.67 (m, 2H) 5.04 (s, 4H) 5.28 (m, 1H) 7.32 (m, 10H) 7.70 (m, 2H)

### EXAMPLE 2

### 2',3'-Dideoxy-3'-fluoro-5'-O-[2,3-bis-(L-valyloxy)propanoyl]guanosine

a) 2', 3'-Dideoxy-3'-fluoro-5'-O-[2,3-bis-(N-CBZ-L-valyloxy)propanoyl]guanosine (MSS-138) A mixture of 2', 3'-dideoxy-3'-fluoroguanosine (2.15g, 8 mmole), 2,3-bis-(N-CBZ-L-valyloxy)-propanoic acid (6.2g, 10.8 mmole), DMAP (244mg, 2 mmole) and HOBT (1.46g, 10.8 mmole) was coevaporated two times with DMF and reduced to about 120ml. DCC (2.48g, 12 mmole) was added and the mixture was stirred for two days at room temperature. The mixture was filtered and the solution was evaporated under reduced pressure. 150ml ethyl acetate was added and the organic phase was washed, twice with 5% acetic acid, with 5% sodium hydrogen carbonate and with water. The organic phase was dried with sodium sulfate and evaporated under reduced pressure. The product was isolated by silica gel column chromatography. Yield: 2,25g = 35%
   ¹H-NMR (DMSO d-6) 0.88 (m,12H) 2,12 (m, 2H) 2.50-3.00 (m, 2H) 3.88-4.14 (m, 2H) 4.22-4.62 (m, 6H) 5.04 (s, 4H) 5.30-5.61 (m, 2H) 6.16 (m, 1H) 6.50 (s, 2H) 7.32 (m, 10H) 7.70 (m, 2H) 7.92 (s, 1H)
b) 2', 3'-Dideoxy-3'-fluoro-5'-O-[2,3-bis-(L-valyloxy)propanoyl] guanosine
   A solution of 2', 3'-dideoxy-3'fluoro-5'-O-[2,3-bis-(N-CBZ-L-valyloxy)propanoyl]guanosine (0.41g, 0.5 mmole) in ethyl acetate (40ml) and acetic acid (20ml) was hydrogenated with palladium black (200mg) at 30 psi for two hours at room temperature. The catalyst was filtered and washed with ethyl acetate and acetic acid. The solution was evaporated under reduced pressure and the product was dried in vacuo to give the dihydrochloride salt. Yield: 0.3g = 95% ¹H-NMR (DMSO d-6 and D₂O) 0.94 (m, 12H) 2.18(m, 2H) 2.52-3.00 (m, 2H) 3.88-4.09 (m, 2H) 4.36-4.72 (m, 6H) 5.42-5.72.

### BIOLOGICAL EXAMPLE 1

### Pharmacokinetics

Confirmation that orally administered prodrugs of the invention release FLG in vivo is obtained in a rat model which is recognized as a useful model for assessing pharmacokinetic parameters of nucleoside analogues. The oral compositions are administered in a pharmaceutical vehicle comprising propylene glycol to triplicate fasted animals in a dosage corresponding to 0.1 mmol/kg. For comparison, a set of rats is iv dosed with 0.01 mmol/kg of the metabolite 2',3'-dideoxy-3'-fluoroguanosine. Serum levels of the metabolite are then monitored in serum collected at intervals from individual animals from 0.5 to up to 6 hours following administration (5 min to 6 hours for FLG).

The metabolite is analysed with HPLC with UV detection at 254 nm, in a manner analogous to Ståhle et al 1995, J Pharm. Biomed. Anal. 13, 369-376. An HPLC system can be based on a 0.05 M ammonium-dihydrogen-phosphate buffer, with 1.2 % 2-propanol solvent, buffered to pH 4.5 or 30 mM sodium dihydrogen phosphate buffer with 2% acetonitrile solvent buffered to pH 7.0. The column may be a 100 x 2.1 mm BAS C18 5 µm particle size with a 7 µm C18 guard column or Zorbax™ SB-CN C18 150x4.6mm, 5µm column. Protein binding of the compounds of the invention is neglible as is that of the metabolite and ultrafiltration through Amicon or Microcon 30 filters is useful for serum samples. Advantageously the main peak is subject to further column chromatography to better aid in resolution of FLG over low weight serum components. The iv levels are multiplied by a factor of ten in order to obtain AUC values for comparison with the oral values. Absolute oral bioavailability is determined as the ratio between ^{0-∞}AUCᵢᵥ and ^{0-∞}AUCₒᵣₐₗ.

The compound of Example 2 showed 0-6 hour absolute bioavailability of 68%, 72% and 64%, resulting in plasma levels of the active metabolite well above the antiviral inhibitory level, as reported in the scientific literature. The compounds of the invention thus provide significantly enhanced oral bioavailability relative to the metabolite metabolite 2',3'-dideoxy-3'-fluoroguanosine. Notably, the compounds are released into the blood in a relatively sustained manner, rather than in an immediate peak. This means that effective amounts of the active metabolite are available in the blood for many hours assisting once daily dosage. Additionally, a sustained release avoids the problems of acute toxicity seen in compounds with a more rapid release rate.

### FORMULATION EXAMPLE 1

### Tablet formulation

The following ingredients are screened through a 0.15 mm sieve and dry-mixed

| | |
|---|---|
| 10 g | 5'-O-[(R)-2,3-bis-(L-valyloxy)-propionyl]-2',3'-dideoxy-3'-fluoro-guanosine, |
| 40 g | lactose |
| 49 g | crystalline cellulose |
| 1 g | magnesium stearate |

A tabletting machine is used to compress the mixture to tablets containing 250 mg of active ingredient.

### FORMULATION EXAMPLE 2

### Enteric coated tablet

The tablets of Formulation Example 1 are spray coated in a tablet coater with a solution comprising

| | |
|---|---|
| 120 g | ethyl cellulose |
| 30 g | propylene glycol |
| 10g | sorbitan monooleate ad 1000 ml aq. dist. |

### FORMULATION EXAMPLE 3

### Controlled release formulation

| | |
|---|---|
| 50 g | 5'-O-[(R)-2,3-bis-(L-valyloxy)-propionyl]-2',3'-dideoxy-3'-fluoroguanosine, |
| 12 g | hydroxypropylmethylcellulose (Methocell™ K15) |
| 4.5 g | lactose |

are dry-mixed and granulated with an aqueous paste of povidone. Magnesium stearate (0.5 g) is added and the mixture compressed in a tabletting machine to 13 mm diameter tablets containing 500 mg active agent.

### FORMULATION EXAMPLE 4

### Soft capsules

| | |
|---|---|
| 250 g | 5'-O-[(S)2,3-bis-(L-valyloxy)-propionyl]-2',3'-dideoxy-3'-fluoroguanosine, |
| 100 g | lecithin |
| 100 g | arachis oil |

The compound of the invention is dispersed in the lecithin and arachis oil and filled into soft gelatin capsules.

## Claims

1. A compound of the formula I 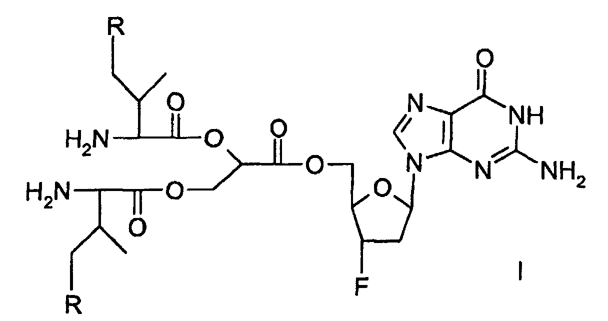 wherein R is independently H or -CH₃;
and pharmaceutically acceptable salts thereof.

2. A compound according to claim 1, wherein both R groups are hydrogen.

3. A compound according to claim 1, selected from the group consisting of
5'-O-[2,3-bis-(L-valyloxy)-propionyl)-2',3'-dideoxy-3'-fluoroguanosine;
5'-O-[2,3-bis-(L-isoleucyloxy)-propionyl]-2',3'-dideoxy-3'-fluoroguanosine;
and their pharmaceutically acceptable salts.

4. A compound according to claim 1, selected from the group consisting of
5'-O-[(R)2,3-bis-(L-valyloxy)-propionyl)-2',3'-dideoxy-3'-fluoroguanosine,
5'-O-[(R)2,3-bis-(L-isoleucyloxy)-propionyl)-2',3'-dideoxy-3'-fluoroguanosine;
and their pharmaceutically acceptable salts.

5. A compound according to claim 2, denoted 5'-O-[(R) 2,3-bis-(L-valyloxy)-propionyl)-2',3'-dideoxy-3'-fluoroguanosine.

6. An anti HIV or anti HBV composition comprising a compound as defined in any preceding claim and a pharmaceutically acceptable carrier therefore.

7. An anti HIV composition according to claim 6, further comprising an additional antiviral selected from AZT (zidovudine), ddI (didanosine), ddC (zalcitabine), d4T (stavudine), 3TC (lamivudine), abacavir, H2G (omaciclovir), ABT 606 (valomaciclovir), foscarnet, ritonavir, indinavir, saquinavir, nevirapine, delaviridine, efavirenz, Vertex VX .478 (amprenavir) or Agouron AG1343 (nelfinavir).

8. An anti HBV composition according to claim 6, further comprising an additional antiviral selected from lamivudine, interferon, famciclovir, adefovir, lobucovir, BMS 200475 (entecavir), L-FMAU (clevudine), FTC (emtricitabine), DAPD (amdoxovir).

9. Use of a compound as defined in any one of claims 1 to 6 in the preparation of a medicament for the treatment of HIV or HBV.

## Patentansprüche

1. Verbindung der Formel I 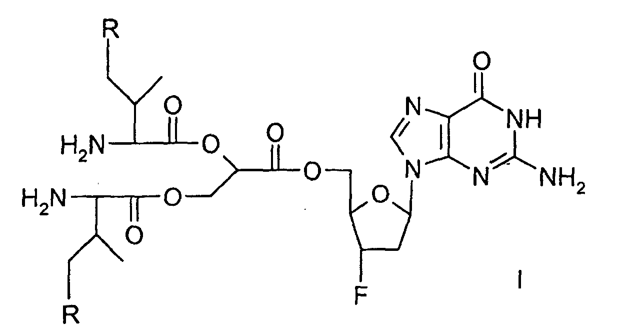 wobei die beiden Reste R unabhängig voneinander H oder -CH₃ sind;
und pharmazeutisch verträgliche Salze dieser Verbindung.

2. Die Verbindung nach Anspruch 1, wobei beide R-Gruppierungen Wasserstoff sind.

3. Die Verbindung nach Anspruch 1, ausgewählt aus der Gruppe bestehend aus
5'-O-[2,3-Bis-(L-valyloxy)-propionyl]-2',3'-didesoxy-3'-fluorguanosin;
5'-O-[2,3-Bis-(L-isoleucyloxy)-propionyl]-2',3'-didesoxy-3'-fluorguanosin;
und deren pharmazeutisch verträgliche Salze.

4. Die Verbindung nach Anspruch 1, ausgewählt aus der Gruppe bestehend aus
5'-O-[(R)2,3-Bis-(L-valyloxy)-propionyl)-2',3'-didesoxy-3'-fluorguanosin,
5'-O-[(R)2,3-Bis-(L-isoleucyloxy)-propionyl]-2',3'-didesoxy-3'-fluorguanosin;
und ihre pharmazeutisch verträglichen Salze.

5. Die Verbindung nach Anspruch 2, wobei diese wie folgt heißt: 5'-O-[(R) 2,3-Bis-(Lvalyloxy)-propionyl]-2',3'-didesoxy-3'-fluorguanosin.

6. Anti-HIV- oder Anti-HBV-Zusammensetzung, umfassend eine Verbindung, definiert wie in einem der vorhergehenden Ansprüche, und einen für diese pharmazeutisch verträglichen Träger.

7. Die Anti-HIV-Zusammensetzung nach Anspruch 6, zusätzlich umfassend ein weiteres antivirales Mittel, ausgewählt aus AZT (Zidovudin), ddI (Didanosin), ddC (Zalcitabin), d4T (Stavudin), 3TC (Lamivudin), Abacavir, H2G (Omaciclovir), ABT 606 (Valomaciclovir), Foscamet, Ritonavir, Indinavir, Saquinavir, Nevirapin, Delaviridin, Efavirenz, Vertex VX 478 (Amprenavir) oder Agouron AG1343 (Nelfinavir).

8. Die Anti-HBV-Zusammensetzung nach Anspruch 6, zusätzlich umfassend ein weiteres antivirales Mittel, ausgewählt aus Lamivudin, Interferon, Famciclovir, Adefovir, Lobucovir, BMS 200475 (Entecavir), L-FMAU (Clevudin), FTC (Emtricitabin), DAPD (Amdoxovir).

9. Verwendung der Verbindung, definiert wie in einem der Ansprüche 1 bis 6, für die Zubereitung eines Medikaments für die Behandlung von HIV oder HBV.

## Revendications

1. Composé de formule I 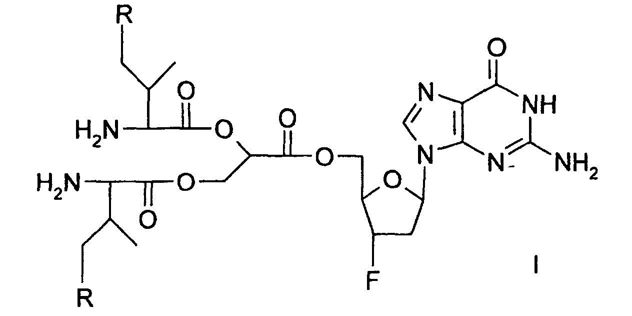 dans laquelle R est indépendamment H ou -CH₃ ;
et ses sels acceptables sur le plan pharmaceutique.

2. Composé selon la revendication 1, dans lequel les deux groupements R sont hydrogène.

3. Composé selon la revendication 1, choisi parmi le groupe constitué de
5'-O-[2,3-bis-(L-valyloxy)-propionyl]-2',3'-didésoxy-3'-fluoroguanosine ;
5'-O-[2,3-bis-(L-isoleucyloxy)-propionyl]-2',3'-didésoxy-3'-fluoroguanosine;
et leurs sels pharmaceutiquement acceptables.

4. Composé selon la revendication 1, choisi parmi le groupe constitué de
5'-O-[(R) 2,3-bis-(L-valyloxy)-propionyl]-2',3'-didésoxy-3'-fluoroguanosine,
5'-O-[(R) 2,3-bis-(L-isoleucyloxy)-propionyl]-2',3'-didésoxy-3'-fluoroguanosine;
et leurs sels pharmaceutiquement acceptables.

5. Composé selon la revendication 2, désigné 5'-O-[(R) 2,3-bis-(L-valyloxy)-propionyl]-2',3'-didésoxy-3'-fluoroguanosine.

6. Composition anti-VIH ou anti-VHB comprenant un composé tel que défini dans l'une des revendications précédentes et un support pharmaceutiquement acceptable de celui-ci.

7. Composition anti-VIH selon la revendication 6, comprenant en outre un antiviral supplémentaire sélectionné parmi l'AZT (zidovudine), le ddI (didanosine), le ddC (zalcitabine), le d4T (stavudine), le 3TC (lamivudine), l'abacavir, le H2G (omaciclovir), l'ABT 606 (valomaciclovir), le foscarnet, le ritonavir, l'indinavir, le saquinavir, la nevirapine, la delaviridine, l'efavirenz, le Vertex VX 478 (amprenavir), ou l'Agouron AG1343 (nelfinavir).

8. Composition anti-HBV selon la revendication 6, comprenant en outre un antiviral supplémentaire sélectionné parmi la lamivudine, l'interféron, le famciclovir, l'adefovir, le lobucovir, le BMS 200475 (entecavir), le L-FMAU (clevudine), le FTC (emtricitabine), le DAPD (amdoxovir).

9. Utilisation d'un composé tel que défini dans l'une des revendications 1 à 6 pour la préparation d'un médicament destiné au traitement du VIH ou VHB.
